# FASCICULE DE BREVET EUROPEEN

(11) **EP 3 752 045 B1**
(45) Date de publication et mention de la délivrance du brevet: **10.11.2021**
(21) Numéro de dépôt: 19711960.5
(22) Date de dépôt: 12.02.2019
(51) Int. Cl.: A61B 3/024, A61B 3/00

(54) **DISPOSITIF DE TEST DE CHAMP VISUEL POUR LA MOBILITE**
SICHTFELDPRÜFVORRICHTUNG FÜR MOBILITÄT
FIELD-OF-VIEW TESTING DEVICE FOR MOBILITY

(30) Priorité: 13.02.2018 FR 1851212
(43) Date de publication de la demande: 23.12.2020
(73) Titulaire: STREETLAB, 75012 Paris (FR); FÉDÉRATION INTERNATIONALE DE L'AUTOMOBILE, 1214 Vernier (CH)
(72) Inventeur: ADRIAN, Julien, 95240 Cormeilles en Parisis (FR); LE BRUN, Johan, 75015 Paris (FR)
(74) Mandataire: Cabinet Netter
(86) Numéro de dépôt international: PCT/FR2019/050299
(87) Numéro de publication internationale: WO 2019/158848

(56) Documents cités:
- WO-A1-91/07908
- WO-A1-2011/132606
- WO-A1-2013/011165
- FR-A1- 3 039 919
- US-A1- 2008 043 201
- US-A1- 2017 049 316

## Description

L'invention concerne le domaine de l'attention visuelle et en particulier les procédés et dispositifs de test du champ visuel pour la mobilité, en particulier la conduite.

Le champ visuel est la portion de l'espace vue par un œil regardant droit devant lui et immobile. La mesure du champ visuel permet donc de quantifier la capacité qu'a une personne à percevoir l'espace qui l'entoure pour une orientation de l'œil.

La conduite est incontestablement une tâche à forte composante visuelle. Au volant d'une automobile, la vision du conducteur et ses capacités d'attention visuelle sont essentielles pour conduire en toute sécurité. La tâche visuelle du conducteur peut être divisée notamment entre le guidage du véhicule (contrôle de la trajectoire) et le balayage visuel de l'environnement pour détecter des événements ou des objets dangereux. Dans ce contexte, les handicaps visuels ou visuo-attentionnels peuvent mener à une incapacité de conduire une voiture en toute sécurité.

Idéalement, l'évaluation des capacités visuelles des conducteurs devrait donc non seulement évaluer les caractéristiques « classiques » de la vision (telle l'acuité visuelle, la sensibilité au contraste, etc.), mais également les capacités visuo-attentionelles et les régulations cognitives (stratégies d'adaptation et de compensation) des conducteurs leur permettant de maintenir une représentation de la scène visuelle routière afin de détecter le plus rapidement possible les changements pouvant survenir dans le champ visuel périphérique.

Pour autant, les études montrent que certaines personnes ayant un déficit visuel ou visuo-attentionnel important semblent ne pas avoir de problèmes en conduite alors que d'autres rencontrent d'importantes difficultés.

Les efforts pour comprendre et évaluer les limitations des conducteurs ont conduit au développement d'un test de champ visuel utile appelé UFOV. Ce test permet d'explorer ce qu'un conducteur peut percevoir dans la zone du champ visuel où l'information peut être acquise en un bref coup d'oeil sans mouvement des yeux ou de la tête (voir par exemple les articles de Ball et al."Age and visual search: expanding the useful field of view", Journal of the Optical Society of America A, 5(12), 2210 et "Visual attention problems as a predictor of vehicle crashes in older drivers", Investigative Ophthalmology & Visual Science, 34(11), 3110-23; Ball, l'article de Sanders "Some Aspects of the Selective Process in the Functional Visual Field", Ergonomics, 13(1), 101-117, et l'article de Scialfa et al."Age differences in target identification as a function of retinal location and noise level: examination of the usefulfield ofview", Psychology and Aging, 2(1), 14-9). Les scores UFOV ont ainsi été mis en lien notamment avec la performance de conduite et le risque d'accident.

Le test UFOV utilise trois tâches visuelles par lesquelles le chercheur évalue le champ « utile » d'un sujet. Dans la première tâche, appelée sous-test 1, le test évalue la capacité du sujet à identifier un stimulus en vision centrale en l'absence d'autres stimuli. La deuxième tâche, appelée sous-test 2, consiste à ajouter simultanément à la première tâche une tâche de localisation périphérique. Enfin, la même double tâche est effectuée (localisation périphérique et identification fovéale), avec l'ajout de stimuli distracteurs (sous-test 3 de l'UFOV).

Les évaluations via l'UFOV ne testent que les cibles périphériques jusqu'à 20-30 d'excentricité maximum, et ne signalent donc pas une mesure de champ utile plus grande que cette étendue testable.

L'un des défauts de ce test est qu'il évalue la capacité des conducteurs à explorer différentes zones du champ visuel sur une étendue limitée, et qu'il ne permet en aucun cas d'évaluer l'ensemble du champ visuel périphérique. Ceci pose problème car d'autres recherches sur le domaine (voir par exemple l'article de Crundall et al. "Driving Experience and the Functional Field of View", Perception, 28(9), 1075-1087) ont permis de montrer que le champ visuel utile n'est pas la seule région d'entrée du traitement visuel. Ainsi, il semble qu'il existe un continuum de perception allant du champ visuel utile au champ visuel périphérique au-delà.

Ces travaux ont donc permis de montrer une diminution de la détectabilité des signaux de danger avec une augmentation de l'excentricité, les auteurs n'ont pas découvert de limite définissant une coupure nette, contrairement à ce qui est suggéré par la restriction au seul champ visuel utile dans le test UFOV.

Le champ visuel utile seul ne permet donc pas de prendre en compte ce que le conducteur peut et ne peut pas percevoir dans son environnement, en particulier à la périphérie. La Demanderesse a donc identifié que le test UFOV n'est pas un bon outil pour tester le champ visuel pour la mobilité, et ne peut pas servir de base de par la dichotomie qu'il impose entre le champ visuel utile et le reste du champ visuel périphérique.

La vision périphérique permet de traiter les scènes visuelles et permet ainsi une compréhension basique d'une scène en moins de 100 ms (soit plus rapidement qu'avec un mouvement des yeux, voir par exemple l'article de Greene et al."The briefest of glances: the time course of natural scene understanding", Psychological Science, 20(4), 464-72). Si quelque chose vient à entrer dans le champ visuel périphérique, le conducteur est donc capable de le détecter très rapidement et d'en tenir compte. De plus la vision périphérique est également efficace pour estimer la valeur caractéristique moyenne (par exemple la taille moyenne) d'un ensemble d'éléments similaires, ou la direction d'un objet mobile. La vision périphérique peut donc être utile au conducteur et délivrer des informations primordiales dans un temps restreint.

De manière beaucoup plus classique et complètement différente du test UFOV, il existe plusieurs types de périmètres pour évaluer l'intégrité du champ visuel clinique. Néanmoins ces tests de périmétrie du champ périphérique ne permettent pas de rendre compte des difficultés de certaines populations, comme par exemple les séniors, dans l'activité de conduite ou une autre activité de mobilité. De plus, toutes les recherches dans ce domaine ont échoué à trouver une relation entre l'état du champ visuel et la difficulté à conduire (telle qu'évaluée par exemple par le taux d'accidents), sauf chez un très faible pourcentage de conducteurs avec une perte de champ binoculaire sévère (voir l'article d'Owsley et al. "Vision and driving", Vision Research).

Ce manque de résultats probant pourrait provenir de l'adaptation tactique des conducteurs, et de l'incapacité de ces systèmes à rendre compte de ces aspects. En effet, ces systèmes imposent au patient de maintenir la tête fixe en la plaçant dans une mentonnière. Ils imposent également de focaliser son regard vers un point de fixation central, tout comme le test UFOV. Or, en conduite, le conducteur à la tête libre et peut organiser son exploration de l'environnement de conduite selon sa volonté. Ces méthodes empêchent donc le conducteur d'adopter des comportements d'autorégulation alors que ceux-ci prévalent lorsque le conducteur est confronté à des difficultés et doit adapter ses comportements à sa déficience. Le document US 2017/049316 A1 qui est considéré comme représentant l'état de la technique le plus proche divulgue un dispositif de test de champ visuel pour la mobilité comprenant un affichage définissant un axe central, une interface agencée pour recevoir des actionnements, et un pilote agencé pour commander l'affichage afin d'afficher une séquence vidéo principale dans laquelle une cible se déplace.

Enfin, les tests de périmètre n'intègrent pas la prise en compte des processus attentionnels du conducteur, qui doit à la fois porter son attention sur la tâche de guidage du véhicule et la détection dans un environnement complexe d'informations périphériques.

L'invention vient améliorer la situation. À cet effet, l'invention propose un dispositif de test de champ visuel pour la mobilité comprenant un affichage définissant un axe central, une interface agencée pour recevoir au moins deux types d'actionnement, et un pilote agencé pour commander l'affichage afin d'afficher une séquence vidéo principale dans laquelle une cible primaire se déplace horizontalement dans un angle de vision compris entre sensiblement -30° et sensiblement +30° par rapport à l'axe central, une marque étant affichée de manière pseudo-aléatoire à l'intérieur de la cible primaire à plusieurs reprises, et une cible secondaire apparaît à des instants choisis à un angle de vision compris entre 60° et 90° en valeur absolue par rapport à l'axe central. Le pilote est en outre agencé pour mesurer et stocker à chaque fois d'une part le temps entre l'affichage d'une marque et l'entrée par un sujet d'un premier type d'actionnement de l'interface, et d'autre part le temps entre l'affichage d'une cible secondaire et l'appui par un sujet d'un second type d'actionnement de l'interface.

Ce dispositif est particulièrement avantageux car il permet de tester l'ensemble du champ visuel d'un sujet pour mesurer sa capacité dans un contexte de mobilité, ce qui n'a pas été possible ou réalisé à ce jour.

Dans divers modes de réalisation, le dispositif pourra présenter une ou plusieurs des caractéristiques suivantes :
- le pilote est agencé pour commander l'affichage afin d'afficher une séquence vidéo principale dans laquelle la cible secondaire apparaît du même côté que la cible primaire par rapport à l'axe central,
- le dispositif comprend en outre un siège sensiblement centré selon l'axe central et propre à recevoir un sujet,
- le pilote est agencé pour commander l'affichage afin d'afficher une séquence vidéo de préparation comprenant uniquement la cible primaire et les marques pseudo-aléatoires et pour mesurer et stocker à chaque fois d'une part le temps entre l'affichage d'une marque et l'entrée par un sujet d'un premier type d'actionnement de l'interface,
- le pilote est agencé pour commander l'affichage avec la séquence vidéo de préparation avant la séquence vidéo principale,
- le pilote est agencé pour commander l'affichage pour afficher la cible secondaire à un angle de vision de -75°, -60°, 60° ou 75° par rapport à l'axe central, et
- le pilote est agencé pour commander l'affichage de chaque cible secondaire pendant 2 secondes.

L'invention concerne également un procédé de test de champ visuel pour la mobilité comprenant les opérations suivantes :
a) Prévoir un affichage définissant un axe central,
b) Positionner un sujet de sorte que le centre de son champ visuel est sensiblement aligné avec l'axe central d'un affichage,
c) Afficher sur l'affichage une séquence vidéo dans laquelle une cible primaire se déplace horizontalement dans un angle de vision compris entre sensiblement -30° et sensiblement +30° par rapport à l'axe central, une marque étant affichée de manière pseudo-aléatoire à l'intérieur de la cible primaire à plusieurs reprises, et une cible secondaire apparaît à des instants choisis à un angle de vision compris entre 60° et 90° en valeur absolue par rapport à l'axe central, et
d) Simultanément à l'opération c), mesurer et stocker à chaque fois d'une part le temps entre l'affichage d'une marque et l'entrée par un sujet d'un premier type d'actionnement de l'interface, et d'autre part le temps entre l'affichage d'une cible secondaire et l'appui par un sujet d'un second type d'actionnement de l'interface.

Dans divers modes de réalisation, le procédé pourra présenter une ou plusieurs des caractéristiques suivantes :
- l'opération c) comprend l'affichage d'une cible secondaire du même côté de l'axe central que la cible primaire,
- le procédé comprend, avant l'exécution des opérations c) et d), les opérations suivantes :
   e) Afficher une séquence vidéo de préparation comprenant uniquement la cible primaire et les marques pseudo-aléatoires, et
   f) Simultanément à l'opération e), mesurer et stocker à chaque fois d'une part le temps entre l'affichage d'une marque et l'entrée par un sujet d'un premier type d'actionnement de l'interface,
- l'opération c) comprend l'affichage de la cible principale à un angle de vision compris entre -30 et 30° par rapport à l'axe central, et
- l'opération c) comprend l'affichage de cibles secondaires à un angle de vision de -75°,
- 60°, 60° ou 75° par rapport à l'axe central.

D'autres caractéristiques et avantages de l'invention apparaîtront mieux à la lecture de la description qui suit, tirée d'exemples donnés à titre illustratif et non limitatif, tirés des dessins sur lesquels :
- la Figure 1 représente une vue schématique d'un dispositif selon l'invention,
- la Figure 2 représente un exemple de mise en œuvre d'une fonction de test du champ visuel pour la mobilité mise en œuvre par l'appareil de la Figure 1,
- la Figure 3 représente un exemple de mise en œuvre d'une autre fonction de test du champ visuel pour la mobilité mise en œuvre par l'appareil de la Figure 1, et
- les Figures 4 à 6 représentent divers écrans vus par un sujet lors de l'exécution des fonctions des Figures 2 et 3.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

La présente description est de nature à faire intervenir des éléments susceptibles de protection par le droit d'auteur et/ou le copyright. Le titulaire des droits n'a pas d'objection à la reproduction à l'identique par quiconque du présent document de brevet ou de sa description, telle qu'elle apparaît dans les dossiers officiels. Pour le reste, il réserve intégralement ses droits.

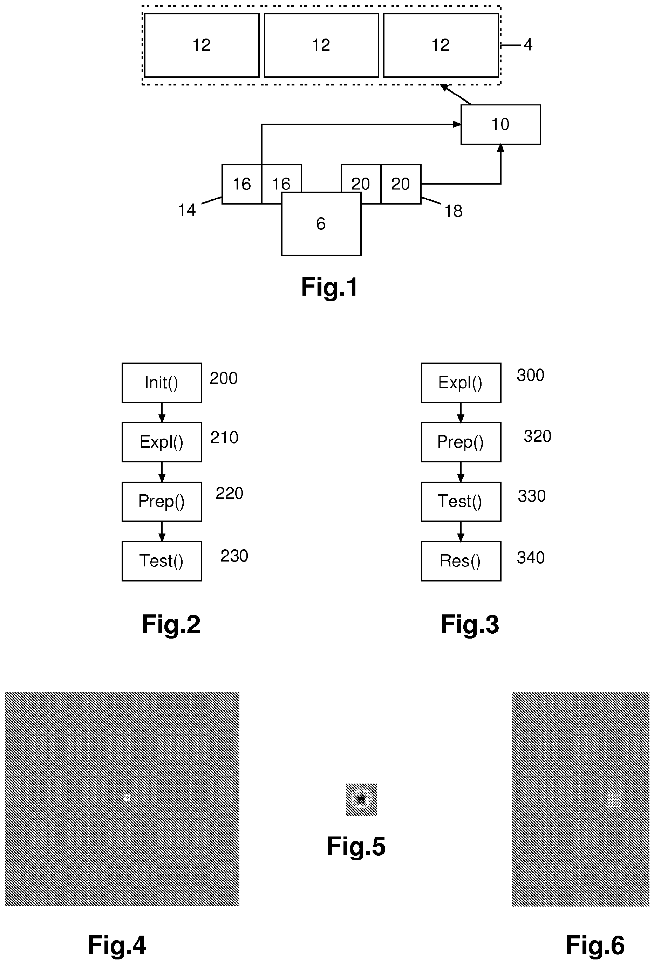

La Figure 1 représente une vue schématique d'un dispositif selon l'invention.

Le dispositif 2 comprend un affichage 4, un siège 6, une interface 8 et un pilote 10.

Dans l'exemple décrit ici, l'affichage 4 comprend trois écrans 12 de type LCD dont la diagonale mesure 65 pouces. Les écrans 12 sont agencés autour du siège 6 de manière à couvrir un champ visuel de 180° pour un sujet installé dans le siège 6. En variante, l'affichage 4 pourra être réalisé différemment, par exemple avec plus d'écrans ou avec des écrans incurvés, d'une technologie différente (OLED ou autre), ou avec un casque de réalité virtuelle. Dans tous les cas, il faut que le centre du champ visuel d'un sujet qui commence un test soit sensiblement aligné avec un axe central défini par l'affichage 4. Dans le cas où le sujet est reçu dans un siège, celui-ci devra être centré par rapport à l'affichage 4. Dans le cas où l'affichage 4 est réalisé au moyen d'un casque de réalité virtuelle, il faudra que celui-ci soit bien centré par rapport aux yeux du sujet pour obtenir le même effet.

Dans l'exemple décrit ici, le siège 6 est un siège de simulation de course automobile. Cela est avantageux car un tel siège est particulièrement adapté à la simulation de la conduite, et car il permet d'intégrer l'interface 8 d'une manière naturelle pour le sujet. En variante, le siège 6 pourrait être réalisé différemment. Pour cela, il conviendrait néanmoins de s'assurer que le sujet garde une liberté de mouvements de la tête et des yeux, et qu'il occupe une position assise comparable à celle d'un conducteur de véhicule.

Dans l'exemple décrit ici, l'interface 8 comprend un pad 14 muni de boutons 16 et un pédalier 18 muni de pédales 20. Plus précisément, dans l'exemple décrit ici, le pad 14 est un pad RB 834 de la société Cedrus (marque déposée) et le pédalier 18 un Club Sport CSP-V3 de la société Fanatec (marque déposée). Comme on le verra par la suite, les boutons 16 et les pédales 20 constituent respectivement un premier type d'actionnement et un deuxième type d'actionnement, qui sont utilisés pour réaliser des mesures distinctes par le pilote 10. En variante, le premier type d'actionnement et le deuxième type d'actionnement pourraient être mis en œuvre uniquement par les boutons 16 ou uniquement par les pédales 20, ou par tout autre type d'actionneur accessible au sujet depuis le siège 6. Par exemple, un unique actionneur pourrait être utilisé, avec la possibilité de l'actionner de deux manières différentes. De même, un volant pourrait être utilisé.

Dans l'exemple décrit ici, le pilote 10 est un ordinateur qui commande l'affichage 4 et reçoit des données d'actionnements de l'interface 8. Le pilote 10 est ici un ordinateur de type PC muni du système d'exploitation Windows 7, d'une carte graphique capable de gérer les écrans 12, reliée au pad 14 et au pédalier 18, et muni d'un écran de contrôle pour les tests réalisés. Bien sûr, le pilote 10 pourra être réalisé de manière différente, avec un système d'exploitation différent, avec une communication avec ou sans fil avec l'affichage 4 et l'interface 8. Par exemple, le pilote 10 pourrait être une tablette ou un smartphone, ou un terminal d'interaction avec un serveur qui est relié à l'affichage 4 et à l'interface 8.

Le dispositif 2 fonctionne comme suit :
- le pilote 10 commande l'affichage 4 pour afficher une ou plusieurs séquences vidéo formant le test à un sujet assis dans le siège 6,
- pendant cette ou ces séquences vidéo, le sujet interagit avec l'interface 8 selon les instructions du test, et
- le pilote 10 reçoit des signaux issus de ces interactions et les met en relation à la ou les séquences vidéo pour tester le champ visuel pour la conduite du sujet.

La Figure 2 représente un exemple d'une première fonction de test mise en œuvre par le pilote 10.

Cette fonction est dans l'exemple décrit ici appelée fonction de préparation, et forme la première partie d'un test en deux parties.

Dans une opération 200, la fonction commence avec l'affichage sur les écrans 12 d'un texte qui explique le déroulement général du test à venir. Par exemple, ce texte peut être : *« Cette évaluation comporte deux parties qui dureront quelques minutes. Lors de chaque partie, une consigne et une phase d'entrainement vous sera présentée. Vous pouvez régler votre siège afin d'être à l'aise pour utiliser la pédale de frein correctement. Au cours du test il vous sera possible de bouger librement votre tête. Lorsque vous êtes prêt(e) appuyez sur le bouton bleu. »*

De manière optionnelle, ce texte peut être traduit dans une autre langue, par exemple l'anglais (ou toute autre langue) et/ou être accompagné d'une restitution sonore.

Ensuite, dans une opération 210, un autre texte peut être affiché pour expliquer le déroulement de la fonction de préparation. Par exemple, ce texte peut être :
*« Dans cette partie, vous devez suivre du regard une cible (un rond gris) et appuyer sur le bouton bleu (bouton de droite) dès qu'une étoile noire apparait au centre de cette cible. Vous devez être le plus rapide possible mais sans vous tromper. Vous devez essayer de détecter toutes les étoiles. Nous allons commencer par quelques essais d'entraînement. Appuyez sur le bouton bleu quand vous êtes prêt(e). »*

De manière optionnelle, ce texte peut être traduit dans une autre langue, par exemple l'anglais (ou toute autre langue) et/ou être accompagné d'une restitution sonore.

L'opération 210 est suivie par une opération 220 dans laquelle le pilote 10 commande l'affichage sur les écrans 12 d'une cible dite principale, sous la forme d'un disque de couleur gris perle, qui se déplace horizontalement, sensiblement au niveau du regard du sujet. De manière pseudo-aléatoire, une marque (ici sous forme d'une étoile noire) est affichée à l'intérieur de la cible primaire, avec optionnellement une indication textuelle ou sonore de l'action à accomplir en réponse (ici appuyer sur l'un des boutons 16).

En variante, certaines ou toutes les opérations 200, 210 et 220 peuvent être omises ou remplacées par une fiche explicative.

La partie préparation est alors lancée dans une opération 230. Dans cette opération, le pilote 10 lance l'affichage d'une séquence vidéo sur les écrans 12. Dans cette séquence vidéo, la cible primaire se déplace horizontalement sur l'écran 12 qui est sensiblement orthogonal à l'axe du siège 6. Dans l'exemple décrit ici, cet écran permet un déplacement de la cible primaire sur un débattement de 60°.

Selon un mode de réalisation préféré de l'invention, le déplacement de la cible primaire correspond au déplacement du regard suivi par une personne pendant une situation de conduite. Par exemple, un pilote automobile peut être filmé pendant une simulation automobile sur un circuit de course automobile ou autre afin de déterminer avec précision le déplacement de son regard. De nombreuses autres méthodes peuvent être employées pour générer la séquence vidéo de déplacement de la cible primaire, et, de manière préférée, le déplacement est réalisé de manière à correspondre au déplacement d'un regard dans une situation de conduite. De nombreux types de mobilité peuvent être utilisés pour servir de base à la génèse de la séquence vidéo, comme la conduite dans un environnement citadin, sur diverses routes, dans des environnements contrôlés comme un aéroport, ou dans d'autres situations de mobilité, etc.

La Figure 4 représente un exemple d'affichage de la cible primaire. Sur cet affichage, la couleur de fond de l'affichage 4 est un gris moyen (code couleur #767676), et la cible primaire une sphère dont la taille correspond à un angle visuel de 1,5° et est de couleur gris perle (code couleur #CECECE). Le choix des nuances de gris pour les stimuli visuels permet de proposer le test même à des personnes souffrant de daltonisme.

Au cours de l'opération 230, le pilote 10 commande l'affichage d'une marque à l'intérieur de la cible primaire. Dès qu'une marque est affichée, le sujet doit activer l'interface 8 et le pilote 10 mesure le temps de réponse du sujet. Lorsque le sujet actionne l'interface 8 pour entrer le premier type d'actionnement, le pilote 10 stocke le temps de réponse et efface la marque.

La Figure 5 représente un exemple d'affichage de la marque. Comme mentionné plus haut, la marque a une forme d'étoile inscrite dans la cible primaire, et présente une couleur noire (code couleur #000000). En variante, la marque peut prendre une autre forme et présenter une couleur différente. Le but est que la marque présente un contraste de luminance par rapport à la couleur de la cible primaire et que la cible primaire est un contraste par rapport à la couleur de fond.

Dans l'exemple décrit ici, tant que le sujet n'entre pas le premier type d'actionnement, la marque reste affichée à l'intérieur de la cible primaire qui se déplace. En variante, la marque pourrait être effacée au bout d'une durée choisie, par exemple au bout de 5 secondes. Dans ce cas, le pilote 10 peut garder une trace des marques qui ont été « ratées » par le sujet, c'est-à-dire pour lesquelles celui-ci n'a pas effectué le premier type d'actionnement. En variante, le pilote 10 peut ignorer les marques ratées.

Dans l'exemple décrit ici, le pilote 10 est également agencé pour déterminer lorsque le premier type d'actionnement est effectué alors qu'il n'y a pas de marque affichée. Dans ce cas, le pilote 10 peut également garder une trace de ces erreurs et/ou indiquer à une personne qui surveille le test lorsqu'un nombre important de telles erreurs est détecté. En effet, certains sujets pourraient être tentés d'effectuer très régulièrement le premier type d'actionnement afin d'optimiser leurs temps de réponse, ce qui fausserait le test. Cette surveillance par le pilote 10 permet de contourner ce problème.

Une fois qu'un sujet a effectué le premier type d'actionnement, ou si le pilote 10 efface la marque, une durée est déterminée de manière pseudo-aléatoire par le pilote 10, et la prochaine marque est affichée à l'expiration de cette durée. Dans l'exemple décrit ici, la durée pseudo-aléatoire est choisie de préférence entre 1 seconde et 5 secondes. En variante, la durée pourrait être choisie entre des bornes plus éloignées ou plus proches, en tenant compte du fait que, si les bornes sont trop éloignées, l'attention du sujet risque d'être diluée, et que si les bornes sont trop proches, le sujet risque d'anticiper l'apparition des marques. En variante, les différentes durées entre l'apparition de deux marques successives pourraient être déterminées à l'avance, avant l'exécution du test. L'opération 230 suit son cours jusqu'à ce que 40 marques aient été affichées. En variante, l'opération 230 peut s'arrêter avant, par exemple quand 20 marques ont été affichées, ou comprendre une autre condition d'arrêt, par exemple basée sur un nombre de marques ratées, sur un nombre d'erreurs détectées, ou sur intervention d'une personne surveillant l'exécution du test.

Enfin la fonction se termine.

La Figure 3 représente un exemple d'une deuxième fonction de test mise en œuvre par le pilote 10.

Cette fonction est dans l'exemple décrit ici appelée fonction principale, et forme la deuxième partie du test en deux parties.

Cette fonction est très proche de la première fonction, sauf en ce que les textes affichés sont différents, et en ce qu'elle ajoute la présence de cibles secondaires et la mesure du temps de réponse de détection de ces dernières.

Ainsi, cette fonction commence par une opération 310 similaire à l'opération 210, mais comprenant l'affichage d'un texte qui décrit le second test. Par exemple, ce texte peut être :
*« Dans ce deuxième test, il vous est demandé de réaliser la même tâche que précédemment. Vous devez appuyer sur le bouton bleu le plus rapidement possible et sans faire d'erreur lorsqu'une étoile apparait. De plus, des cibles en mouvement apparaîtront à la périphérie (d'un seul côté pour l'entrainement). Vous devrez alors appuyer sur la pédale de frein le plus rapidement possible et sans faire d'erreur lorsque vous les percevrez. La tâche de détection de cible étoile est prioritaire mais vous devez essayer de détecter le plus de cible périphérique possible. Appuyez sur le bouton bleu quand vous êtes prêt(e). »*

De manière optionnelle, ce texte peut être traduit dans une autre langue, par exemple l'anglais (ou toute autre langue) et/ou être accompagné d'une restitution sonore. Ensuite, dans une opération 320, une séquence de tests similaire à l'opération 220 est exécutée. Dans cette opération, le pilote 10 commande l'affichage 4 pour faire apparaître de manière pseudo-aléatoire des cibles secondaires sur les écrans 12. Les cibles secondaires sont situées sur un plan horizontal sensiblement à la même hauteur que la cible primaire, mais elles sont beaucoup plus excentrées. En effet, les cibles secondaires apparaissent à l'extrémité gauche ou droite de l'affichage 4, et se déplacent jusqu'à un angle de 75° ou 60° en valeur absolue par rapport à l'axe central défini par l'affichage 4. Ce déplacement est ici réalisé à vitesse constante, et, une fois arrêtée, chaque cible secondaire reste affichée pendant 2 secondes. Pour montrer qu'il a détecté une cible secondaire, le sujet doit entrer le deuxième type d'actionnement, c'est-à-dire appuyer sur les pédales 20 dans l'exemple décrit ici.

Le fait d'afficher les cibles secondaires à un angle de 60° ou 75° permet de tester le champ visuel périphérique, et le déplacement des cibles secondaires peut correspondre à l'apparition d'un véhicule qu'il est crucial de détecter.

En variante, les cibles secondaires pourraient se déplacer jusqu'à un angle aléatoirement choisi entre 60° et 75°. De plus, le déplacement depuis le bord de l'affichage 4 pourrait avoir une vitesse variable, ou les cibles secondaires pourraient être affichées directement à leur position d'arrêt sans déplacement. Au lieu d'un déplacement des cibles secondaires selon une translation, celles-ci pourraient être remplacées par une apparition progressive par grossissement à partir d'une apparition initiale de plus petite dimension. Les translations pourraient également être modifiées de sorte que les points de départ des cibles secondaires (le bord de l'affichage 4) et d'arrivée (à 60° ou 75° de l'axe central) soient différents et plus variés, voire aléatoires. La direction des translations pourrait également être modifiée - une ou plusieurs ou toutes pourraient être de nature verticale et non pas horizontale. Comme pour l'opération 220, le sujet peut être assisté par un affichage et/ou des consignes sonores. Cette opération peut également comprendre une calibration de la hauteur d'affichage des cibles secondaires, afin que les branches de lunettes du sujet ne les masquent pas.

La Figure 6 représente un exemple d'affichage de la cible secondaire. Sur cet affichage, la couleur de fond de l'affichage 4 est toujours un gris moyen (code couleur #767676), et la cible secondaire un rectangle dont la taille correspond à un angle visuel de 3° et est de couleur gris perle (code couleur #CECECE). Ici encore, un contraste de luminance est recherché avec la couleur du fond, mais la cible secondaire pourrait prendre une forme différente et/ou une couleur différente.

Une fois cet entraînement réalisé, une opération 330 est réalisée. L'opération 330 comprend l'intégralité de l'opération 230, sauf que l'apparition de cibles secondaires et la mesure du temps de détection par le sujet lui est ajoutée.

Dans l'exemple décrit ici, le moment auquel apparaissent les cibles secondaires peut être fixé afin de correspondre à un moment auquel un véhicule serait susceptible d'apparaître dans la simulation qui a servi à élaborer le déplacement de la cible primaire. En variante, l'apparition des cibles secondaires pourrait être réalisée de manière pseudo-aléatoire, comme pour les marques. Dans l'exemple décrit ici, l'opération 330 est donc un cycle d'apparition de marques et de cibles secondaires, avec 40 cibles secondaires, dont 10 s'arrêtent à 60° par rapport à l'axe central sur la partie côté gauche de l'affichage 4, 10 s'arrêtent à 75° par rapport à l'axe central sur la partie côté gauche de l'affichage 4, 10 s'arrêtent à 60° par rapport au sujet sur la partie côté gauche de l'affichage 4, et 10 s'arrêtent à 75° par rapport au sujet sur la partie côté gauche de l'affichage 4. Le choix de la valeur de l'angle d'arrêt est ici aléatoire, de sorte qu'une cible secondaire apparaît du côté de l'affichage où est située la cible primaire, mais avec une valeur de 60° ou de 75° choisie aléatoirement (dans le respect des groupes de 10 tests pour chaque valeur d'arrêt décrits plus haut). En variante, le nombre de cibles et leurs angles d'arrêts pourraient être changés.

Si un sujet n'entre pas le deuxième type d'actionnement pendant la durée d'affichage de la cible secondaire correspondante, le pilote 10 note cette absence de réponse. En variante, l'absence de réponse pourrait ne pas être notée. Toujours en variante, le pilote 10 peut également noter la présence de déclenchement imprévu de l'interface 8. Ainsi, comme pour le premier type d'interaction, il serait possible de détecter un sujet qui active de manière répétée l'interface 8 en l'absence de stimulus afin de « deviner » ou d'anticiper la survenue d'un stimulus.

Une fois l'opération 330 terminée, le pilote 10 restitue les résultats résumant les temps de réponse liés à la cible primaire, et les temps de réponse liés aux cibles secondaires, ainsi que les erreurs. De manière préférée, le pilote 10 indique la moyenne et la variance pour chaque type de mesure, et sépare les réponses relatives aux cibles secondaires en fonction de l'angle d'arrêt (60° ou 75°) et du côté de l'affichage concerné. De plus, le pilote 10 peut accéder à une base de données de référence afin de rapprocher le sujet d'un groupe de sujets pertinents, que ce soit par âge, pathologie (ou absence de pathologie), sexe, ou tout autre critère pertinent, et indiquer à quel percentile le sujet appartient par rapport au groupe auquel il appartient.

La lecture de ces résultats par un praticien, par exemple un médecin ophtalmologue peut lui permettre de qualifier le champ visuel de conduite du sujet, et émettre une recommandation quant à l'aptitude de ce dernier à conduire.

Dans ce qui précède, un test à deux étapes a été décrit. En variante, un test pourrait être réduit à l'utilisation de la fonction de la Figure 3 uniquement.

Bien que ce qui précède ait été décrit relativement à la conduite automobile, il apparaît qu'il pourrait être généralisé à d'autres types de conduites, par exemple nautique ou aérienne, d'autres types de véhicules comme une moto ou autre, et plus généralement à toute mobilité dans laquelle la vision périphérique est cruciale, comme par exemple le traitement de joueurs de sports comme le football américain ou le rugby ou autre qui ont subi un traumatisme. De fait, le dispositif et le procédé de l'invention peuvent être intégrés dans de nombreuses applications de qualification de sujet pour la pratique d'activités dans lesquelles le champ visuel périphérique présente une importance.

## Revendications

1. Dispositif de test de champ visuel pour la mobilité comprenant un affichage (4) définissant un axe central, une interface (8) agencée pour recevoir au moins deux types d'actionnement (16, 20), et un pilote (10) agencé pour commander l'affichage (4) afin d'afficher une séquence vidéo principale dans laquelle une cible primaire se déplace horizontalement dans un angle de vision compris entre sensiblement -30° et sensiblement +30° par rapport à l'axe central, une marque étant affichée de manière pseudo-aléatoire à l'intérieur de la cible primaire à plusieurs reprises, et une cible secondaire apparaît à des instants choisis à un angle de vision compris entre 60° et 90° en valeur absolue par rapport à l'axe central, le pilote (10) étant en outre agencé pour mesurer et stocker à chaque fois d'une part le temps entre l'affichage d'une marque et l'entrée par un sujet d'un premier type d'actionnement de l'interface (8), et d'autre part le temps entre l'affichage d'une cible secondaire et l'appui par un sujet d'un second type d'actionnement de l'interface (8).

2. Dispositif selon la revendication 1, dans lequel le pilote (10) est agencé pour commander l'affichage (4) afin d'afficher une séquence vidéo principale dans laquelle la cible secondaire apparaît du même côté que la cible primaire par rapport à l'axe central.

3. Dispositif selon la revendication 1 ou 2, comprenant en outre un siège (6) sensiblement centré selon l'axe central et propre à recevoir un sujet.

4. Dispositif selon l'une des revendications précédentes, dans lequel le pilote (10) est agencé pour commander l'affichage (4) afin d'afficher une séquence vidéo de préparation comprenant uniquement la cible primaire et les marques pseudo-aléatoires et pour mesurer et stocker à chaque fois d'une part le temps entre l'affichage d'une marque et l'entrée par un sujet d'un premier type d'actionnement de l'interface (8).

5. Dispositif selon la revendication 4, dans lequel le pilote (10) est agencé pour commander l'affichage (4) avec la séquence vidéo de préparation avant la séquence vidéo principale.

6. Dispositif selon l'une des revendications précédentes, dans lequel le pilote (10) est agencé pour commander l'affichage (4) pour afficher la cible secondaire à un angle de vision de -75°, -60°, 60° ou 75° par rapport à l'axe central.

7. Dispositif selon la revendication 6, dans lequel le pilote (10) est agencé pour commander l'affichage (4) de chaque cible secondaire pendant 2 secondes.

8. Procédé de test de champ visuel pour la mobilité comprenant les opérations suivantes :
a) Prévoir un affichage (4) définissant un axe central,
b) Positionner un sujet de sorte que le centre de son champ visuel est sensiblement aligné avec l'axe central d'un affichage (4),
c) Afficher sur l'affichage (4) une séquence vidéo dans laquelle une cible primaire se déplace horizontalement dans un angle de vision compris entre sensiblement -30° et sensiblement +30° par rapport à l'axe central, une marque étant affichée de manière pseudo-aléatoire à l'intérieur de la cible primaire à plusieurs reprises, et une cible secondaire apparaît à des instants choisis à un angle de vision compris entre 60° et 90° en valeur absolue par rapport à l'axe central, et
d) Simultanément à l'opération c), mesurer et stocker à chaque fois d'une part le temps entre l'affichage d'une marque et l'entrée par un sujet d'un premier type d'actionnement de l'interface (8), et d'autre part le temps entre l'affichage d'une cible secondaire et l'appui par un sujet d'un second type d'actionnement de l'interface (8).

9. Procédé selon la revendication 8, dans lequel l'opération c) comprend l'affichage d'une cible secondaire du même côté de l'axe central que la cible primaire.

10. Procédé selon la revendication 8 ou 9, comprenant, avant l'exécution des opérations c) et d), les opérations suivantes :
e) Afficher une séquence vidéo de préparation comprenant uniquement la cible primaire et les marques pseudo-aléatoires, et
f) Simultanément à l'opération e), mesurer et stocker à chaque fois d'une part le temps entre l'affichage d'une marque et l'entrée par un sujet d'un premier type d'actionnement de l'interface (8).

11. Procédé selon l'une des revendications 8 à 10, dans lequel l'opération c) comprend l'affichage de la cible principale à un angle de vision compris entre -30 et 30° par rapport à l'axe central.

12. Procédé selon l'une des revendications 8 à 11, dans lequel l'opération c) comprend l'affichage de cibles secondaires à un angle de vision de -75°, -60°, 60° ou 75° par rapport à l'axe central.

## Patentansprüche

1. Vorrichtung zur Prüfung des Gesichtsfeldes für die Mobilität, welche eine Anzeige (4), die eine zentrale Achse definiert, eine Schnittstelle (8), die so eingerichtet ist, dass sie mindestens zwei Betätigungsarten (16, 20) aufnimmt, und einen Treiber (10) umfasst, der so eingerichtet ist, dass er die Anzeige (4) steuert, um eine Haupt-Videosequenz anzuzeigen, in der sich ein primäres Ziel horizontal in einem Sichtwinkel im Bereich zwischen im Wesentlichen -30° und im Wesentlichen +30° in Bezug auf die zentrale Achse bewegt, wobei mehrmals pseudozufällig eine Markierung innerhalb des primären Ziels angezeigt wird und zu ausgewählten Zeitpunkten ein sekundäres Ziel in einem Sichtwinkel im Bereich zwischen 60° und 90° Absolutwert in Bezug auf die zentrale Achse erscheint, wobei der Treiber (10) weiter so eingerichtet ist, dass er jedes Mal einerseits die Zeit zwischen der Anzeige einer Markierung und der Eingabe einer ersten Betätigungsart der Schnittstelle (8) durch einen Probanden, und andererseits die Zeit zwischen der Anzeige eines sekundären Ziels und dem Drücken einer zweiten Betätigungsart der Schnittstelle (8) durch einen Probanden misst und speichert.

2. Vorrichtung nach Anspruch 1, wobei der Treiber (10) so eingerichtet ist, dass er die Anzeige (4) steuert, um eine Haupt-Videosequenz anzuzeigen, in der das sekundäre Ziel in Bezug auf die zentrale Achse auf der gleichen Seite erscheint wie das primäre Ziel.

3. Vorrichtung nach Anspruch 1 oder 2, die weiter einen Sitz (6) umfasst, der im Wesentlichen auf der zentralen Achse zentriert und dazu geeignet ist, einen Probanden aufzunehmen.

4. Vorrichtung nach einem der vorstehenden Ansprüche, wobei der Treiber (10) so eingerichtet ist, dass er die Anzeige (4) steuert, um eine Vorbereitungs-Videosequenz anzuzeigen, die ausschließlich das primäre Ziel und die pseudozufälligen Markierungen umfasst, und jedes Mal einerseits die Zeit zwischen der Anzeige einer Markierung und der Eingabe einer ersten Betätigungsart der Schnittstelle (8) durch einen Probanden misst und speichert.

5. Vorrichtung nach Anspruch 4, wobei der Treiber (10) so eingerichtet ist, dass er die Anzeige (4) mit der Vorbereitungs-Videosequenz vor der Haupt-Videosequenz steuert.

6. Vorrichtung nach einem der vorstehenden Ansprüche, wobei der Treiber (10) so eingerichtet ist, dass er die Anzeige (4) steuert, um das sekundäre Ziel in einem Sichtwinkel von -75°, -60°, 60° oder 75° in Bezug auf die zentrale Achse anzuzeigen.

7. Vorrichtung nach Anspruch 6, wobei der Treiber (10) so eingerichtet ist, dass er die Anzeige (4) jedes sekundären Ziels während 2 Sekunden Steuer.

8. Verfahren zur Prüfung des Gesichtsfeldes für die Mobilität, das die folgenden Vorgänge umfasst:
a) Vorsehen einer Anzeige (4), die eine zentrale Achse definiert,
b) Positionieren eines Probanden so, dass das Zentrum seines Gesichtsfeldes im Wesentlichen mit der zentralen Achse einer Anzeige (4) fluchtet,
c) Anzeigen einer Videosequenz auf der Anzeige (4), in der sich ein primäres Ziel horizontal in einem Sichtwinkel im Bereich zwischen im Wesentlichen -30° und im Wesentlichen +30° in Bezug auf die zentrale Achse bewegt, wobei mehrmals pseudozufällig eine Markierung innerhalb des primären Ziels angezeigt wird, und zu ausgewählten Zeitpunkten ein sekundäres Ziel in einem Sichtwinkel im Bereich zwischen 60° und 90° Absolutwert in Bezug auf die zentrale Achse erscheint, und
d) gleichzeitig mit Vorgang c), jedes Mal Messen und Speichern einerseits der Zeit zwischen der Anzeige einer Markierung und der Eingabe einer ersten Betätigungsart der Schnittstelle (8) durch einen Probanden, und andererseits der Zeit zwischen der Anzeige eines sekundären Ziels und dem Drücken einer zweiten Betätigungsart der Schnittstelle (8) durch einen Probanden.

9. Verfahren nach Anspruch 8, wobei der Vorgang c) das Anzeigen eines sekundären Ziels auf der gleichen Seite der zentralen Achse wie das primäre Ziel umfasst.

10. Verfahren nach Anspruch 8 oder 9, das vor dem Ausführen der Vorgänge c) und d) die folgenden Vorgänge umfasst:
e) Anzeigen einer Vorbereitungs-Videosequenz, die ausschließlich das primäre Ziel und die pseudozufälligen Markierungen umfasst, und
f) gleichzeitig mit Vorgang e), jedes Mal Messen und Speichern einerseits der Zeit zwischen der Anzeige einer Markierung und der Eingabe einer ersten Betätigungsart der Schnittstelle (8) durch einen Probanden.

11. Verfahren nach einem der Ansprüche 8 bis 10, wobei der Vorgang c) das Anzeigen des Hauptziels in einem Sichtwinkel im Bereich zwischen -30 und 30° in Bezug auf die zentrale Achse umfasst.

12. Verfahren nach einem der Ansprüche 8 bis 11, wobei der Vorgang c) das Anzeigen von sekundären Zielen in einem Sichtwinkel von -75°, -60°, 60° oder 75° in Bezug auf die zentrale Achse umfasst.

## Claims

1. A field-of-view testing device for mobility comprising a display (4) defining a central axis, an interface (8) arranged to receive at least two types of actuation (16, 20), and a driver (10) arranged to control the display (4) in order to display a main video sequence wherein a primary target moves horizontally within a viewing angle between substantially -30° and substantially +30° relative to the central axis, a mark being pseudo-randomly displayed within the primary target several times, and a secondary target appears at selected times at a viewing angle between 60° and 90° in absolute value relative to the central axis, the driver (10) being furthermore arranged to measure and store each time, on the one hand, the time between a mark being displayed and a subject inputting a first type of actuation of the interface (8), and on the other hand, the time between a secondary target being displayed and a subject pressing a second type of actuation of the interface (8).

2. The device according to claim 1, wherein the driver (10) is arranged to control the display (4) in order to display a main video sequence wherein the secondary target appears on the same side as the primary target relative to the central axis.

3. The device according to claim 1 or 2, further comprising a seat (6) substantially centred along the central axis and suitable for receiving a subject.

4. The device according to one of the preceding claims, wherein the driver (10) is arranged to control the display (4) in order to display a preparation video sequence comprising only the primary target and the pseudo-random marks and to measure and store each time, on the one hand, the time between a mark being displayed and a subject inputting a first type of actuation of the interface (8).

5. The device according to claim 4, wherein the driver (10) is arranged to control the display (4) with the preparation video sequence before the main video sequence.

6. The device according to one of the preceding claims, wherein the driver (10) is arranged to control the display (4) to display the secondary target at a viewing angle of -75°, -60°, 60° or 75° relative to the central axis.

7. The device according to claim 6, wherein the driver (10) is arranged to control the display (4) of each secondary target for 2 seconds.

8. A field-of-view testing method for mobility comprising the following operations:
a) Providing a display (4) defining a central axis,
b) Positioning a subject so that the centre of its field of view is substantially aligned with the central axis of a display (4),
c) Displaying on the display (4) a video sequence wherein a primary target moves horizontally within a viewing angle between substantially -30° and substantially +30° relative to the central axis, a mark being pseudo-randomly displayed within the primary target repeatedly, and a secondary target appears at selected times at a viewing angle between 60° and 90° in absolute value relative to the central axis, and
d) Simultaneously with the operation c), measuring and storing each time, on the one hand, the time between a mark being displayed and a subject inputting a first type of actuation of the interface (8), and on the other hand, the time between a secondary target being displayed and a subject pressing a second type of actuation of the interface (8).

9. The method according to claim 8, wherein the operation c) comprises displaying a secondary target on the same side of the central axis as the primary target.

10. The method according to claim 8 or 9, comprising, before the execution of operations c) and d), the following operations:
e) Displaying a preparation video sequence comprising only the primary target and the pseudo-random marks, and
f) Simultaneously with operation e), measuring and storing each time, on the one hand, the time between a mark being displayed and a subject inputting a first type of actuation of the interface (8).

11. The method according to one of claims 8 to 10, wherein operation c) comprises displaying the main target at a viewing angle between -30 and 30° relative to the central axis.

12. The method according to one of claims 8 to 11, wherein the operation c) comprises displaying secondary targets at a viewing angle of -75°, -60°, 60° or 75° relative to the central axis.
